# EUROPEAN PATENT SPECIFICATION

(11) **EP 3 617 325 B1**
(45) Date of publication and mention of the grant of the patent: **20.10.2021**
(21) Application number: 19194184.8
(22) Date of filing: 28.08.2019
(51) Int. Cl.: C12Q 1/6841

(54) **RNA-GUIDED ENDONUCLEASE DNA LABELING**
RNA-GESTEUERTE ENDONUKLEASE-DNA-MARKIERUNG
ÉTIQUETAGE D'ADN D'ENDONUCLÉASES GUIDÉES PAR ARN

(30) Priority: 29.08.2018 DE 102018121111; 29.08.2018 LU 100916
(43) Date of publication of application: 04.03.2020
(73) Proprietor: Leibniz-Institut für Pflanzengenetik und Kulturpflanzenforschung (IPK), 06466 Gatersleben (DE)
(72) Inventor: Ishii, Takayoshi, Tottori-shi, 6800901 (DE); Houben, Andreas, 06484 Quedlinburg (DE)
(74) Representative: Stüven, Ralf

(56) References cited:
- WO-A1-2016/061523
- WO-A1-2017/053762
- WO-A1-2018/111946
- Denghong Zhang ET AL: "CRISPR-bind: a simple, custom CRISPR/dCas9-mediated labeling of genomic DNA for mapping in nanochannel arrays", bioRxiv, 19 July 2018 (2018-07-19), XP055586613, DOI: 10.1101/371518 Retrieved from the Internet: URL:https://www.biorxiv.org/content/biorxi v/early/2018/07/19/371518.full-text.pdf

## Description

The invention relates to a method for the detection of a target DNA sequence in a sample.

In situ hybridization is a powerful tool to physically map DNA sequences for research and diagnostic purposes. One major disadvantage of this method is the need of any kind of DNA denaturation to achieve complementary base pairing in double-stranded DNA. Such harsh treatment invariably degrades the structure of the specimen (Boettiger et al., 2016; Kozubek et al., 2000). Further, besides denaturation, classical FISH includes hybridization and post-hybridization washes, which require hours to days to receive a final result.

The discovery of the type II clustered regularly interspaced short palindromic repeats (CRISPR)-associated caspase 9 (Cas9) system derived from Streptococcus pyogenes as a programmable endonuclease, revolutionized the field of targeted genome editing in eukaryotes (Jinek et al., 2012). The detection of specific sequences by CRISPR/Cas9 occurs without global DNA denaturation. The Cas9-RNA complex first recognizes a short nucleotide sequence (NGG for S. pyogenes Cas9) 3' to a target sequence in the dsDNA, called a protospacer adjacent motif (PAM). Then, Cas9-RNA initiates DNA unwinding at the PAM-proximal region, followed by the directional formation of an R-loop, consisting of the RNA-DNA hybrid and the displaced non-target DNA strand (Sternberg et al., 2014).

Cas9 nuclease based genome engineering has become a routine technology for many species (reviewed in Ahmad et al., 2018). However, the full potential of this technology reaches far beyond the controlled induction of mutations (Wang et al., 2016). Recently, nuclease-deficient derivatives were used to visualize genomic loci in living (Anton et al., 2014; Chen et al., 2013; Dreissig et al., 2017) and fixed cells (Deng et al., 2015). In contrast to classical FISH, CRISPR-Cas9-mediated in situ labeling of genomic loci in fixed cells does not require DNA denaturation and permits therewith a better structural preservation of the specimen.

Zhang et al. (2018) describe the use of nuclease-deficient dCas9 with no cutting activity to directly label DNA with a fluorescent CRISPR-dCas9 complex composed of a dCas9 protein, a custom target crRNA and a fluorescently labeled tracrRNA, in genome mapping.

The use of fluorophore-coupled sgRNA in combination with fluorophore as well as HaloTag coupled nuclease-deficient Cas9 (dCas9) termed Cas9-mediated FISH (CAS-FISH) allowed the labeling of repetitive DNA elements in fixed mammalian cells (Deng et al., 2015). However, further optimization of this technology is needed before it can be used for routine applications. In particular, the development of a technique that is simple, and does not require extensive sample preparation remains highly desirable.

WO 2017/053762 A1 describes methods and reagents for molecular proximity detection using RNA-guided nucleic acid binding proteins. A complex is described comprising at least one probe, the probe comprising an engineered, non-naturally occurring CRISPR/Cas system comprising a CRISPR/Cas protein and a gRNA, and an oligonucleotide directly or indirectly linked to the CRISPR/Cas system.

It is an object of the invention to provide a robust and reliable method for detecting specific DNA sequences in a tissue sample, which is simple and does not require extensive sample preparation.

To solve the above problem the invention provides a method for the detection of a target DNA sequence in a sample, the sample being a tissue section which has been fixed with a buffered 4% paraformaldehyde solution, wherein the sample is contacted with a ribonucleoprotein complex comprising an unlabeled Cas protein, an unlabeled crRNA hybridizing with the target DNA sequence and a labeled tracrRNA hybridizing with the crRNA under conditions where the target DNA in the sample is able to hybridize with the crRNA, and wherein the paraformaldehyde-fixed tissue section is contacted with a buffered 4% paraformaldehyde solution under cooling after being contacted with the ribonucleoprotein complex.

The method of the invention is robust, versatile, simple and comparatively fast. In particular, it does not require extensive sample preparation. It is especially useful in detecting chromosomal and nuclear DNA sequences in tissue sections. The method of the invention can easily be combined with immunostaining of proteins and thus can be used to simultaneously detect proteins, e.g. DNA binding proteins, via labeled antibodies. DNA denaturation is not required and thus the use of hazardous formamide, which is used for in situ denaturation of DNA, can be avoided. In addition, any necessary washing steps are less time-consuming.

In the method of the invention a sample a tissue sample, which has been fixed with a buffered 4% paraformaldehyde solution, is contacted with a ribonucleoprotein (RNP) complex, the RNP complex comprising an unlabeled Cas protein, an unlabeled crRNA, which hybridizes with the target DNA sequence, and a labeled tracrRNA, which hybridizes with the crRNA, under conditions where a target DNA present in the sample is able to hybridize with the crRNA. The paraformaldehyde-fixed tissue section is contacted with a buffered 4% paraformaldehyde solution under cooling after being contacted with the RNP complex. The RNP complex forms a complex with the target DNA sequence by binding to the labeled tracrRNA, and the formed RNP-DNA complex can be detected via the label, e.g. a fluorophore, attached to the tracrRNA. The target DNA can thus be detected and visualized in the sample.

The term "sample" means any material, in particular biological material, to be tested for the presence, absence and/or quantity of a DNA sequence. A "biological sample" is understood to mean a sample of biological material, in particular a sample comprising cell or tissue material, for example a tissue section, a cell smear, a chromosome or nuclei suspension. The sample can be fixed on a support, e.g. a microscope slide. The sample can be, for example, of plant, animal or human origin.

The term "ribonucleoprotein complex" or "RNP" complex means a complex comprising or consisting of one or more proteins or peptides and one or more ribonucleic acid (RNA) molecules.

The term "Cas protein" refers to a CRISPR associated protein. CRISPR is an abbreviation for CRISPR is an abbreviation for the term "Clustered Regularly Interspaced Short Palindromic Repeats" used to denote sections of prokaryotic DNA containing palindromic repeat sequences, interspaced by segments of variable spacer DNA. The term encompasses a family of proteins being part of the CRISPR-Cas system, which is divided in two classes (class 1 and 2) and several types (I to VI) and subtypes. Class 1 encompasses types I, III, and IV, class 2 types II, V, and VI. The Cas types are often characterized by a signature protein. Cas9, for example, is a signature protein for Cas type II. See, e.g., Makarova et al. 2015; Koonin et al. 2017; Haft et al. 2005, Wright et al. 2016. Cas proteins have nuclease activity and use CRISPR RNAs (crRNAs) to guide a Cas nuclease component to the target nucleic acid molecule to be cleaved.

The term "Cas9 protein" or "Cas9" refers to the CRISPR associated protein 9. Cas9 is an RNA-guided DNA endonuclease enzyme, e.g. from *Streptococcus pyogenes.* The term "Cas9" as used herein also encompasses all Cas9 orthologs and also recombinant, i.e. engineered variants thereof, e.g. partially or entirely nuclease deficient Cas9 proteins (nickase Cas9 = nCas9, nuclease deficient Cas9 = dCas9). The Cas9 nuclease is active when it forms a complex with two RNA molecules, the tracrRNA and the crRNA. Part of the crRNA sequence defines the specificity of the nuclease by complementary base pairing with the target DNA sequence. By specifying the targeting sequence of the crRNA it is possible to direct the CRISPR-Cas9 system to the appropriate target site. A further requirement for Cas9-mediated DNA targeting is the presence of a short (e.g. 2-6 base pairs) and conserved protospacer adjacent motif (PAM) flanking the target site. The PAM sequence can be adapted by engineering the Cas protein.

The term "crRNA" refers to CRISPR RNA, and refers to an RNA molecule being able to complement with a tracrRNA. The crRNA confers target specificity to a Cas protein, e.g. Cas9. The RNA:RNA duplex composed of crRNA and tracrRNA is also called guide RNA (gRNA) and binds to the Cas protein. In bacterial CRISPR loci, the crRNA is found in the CRISPR repeat/spacer section and consists of the spacer, which is complementary to the target, and the repeat that complements with the tracrRNA.

The term "tracrRNA" refers to trans-activating RNA, a small trans-encoded RNA that is partially complementary to and base pairs with crRNA, thus forming an crRNA:tracrRNA duplex.

The term "guide RNA", gRNA, refers to a crRNA:tracrRNA duplex. If crRNA and tracrRNA are fused to a single RNA molecule, the term "single guide RNA" (sgRNA) may also be used.

The term "hybridize" relates to the formation of a duplex by base pairing between complementary nucleic acid molecules, e.g. between two RNA molecules or an RNA and a DNA molecule.

The term "labeled" means attaching a label to a substance, molecule, or cell in order to make it identifiable by optical or other means. A label can, for example, be attached to a molecule by replacing an atom with another atom, chemical group or molecule, e.g. with a radioactive isotope, a fluorescent dye, enzyme, or other molecule.

The term "under conditions where the target DNA in the sample is able to hybridize with the crRNA" relates to conditions, e.g. in terms of temperature, pH, salt concentration etc., under which the target DNA can form a duplex, i.e. can base pair with the complementary part of the crRNA. Those conditions are known to the skilled person.

In a preferred embodiment of the method of the invention the crRNA and the tracrRNA are separate RNA molecules. It is possible to use a single RNA molecule composed of the crRNA and the tracrRNA, i.e. a single guide RNA (sgRNA), in the method of the invention. However, it is preferred to use a "two-part" guide RNA, i.e. a duplex of two separate RNA molecules.

In a preferred embodiment of the method of the invention the Cas protein is a recombinant Cas protein, preferably a recombinant type II Cas protein, e.g. a recombinant Cas9 protein, further preferred a Cas protein lacking nuclease activity, especially preferred a nCas9 protein or dCas9 protein.

In a further preferred embodiment of the method of the invention the crRNA and the tracrRNA are synthetic RNAs, and the crRNA and the tracrRNA are preferably each shorter than naturally occurring crRNAs and tracrRNAs. Preferably, the synthetic crRNA and tracrRNA are chemically modified, e.g. by replacing nucleotides naturally occurring in crRNA and tracrRNA with nucleotides not naturally occurring in crRNA and tracrRNA, e.g. in order to stabilize the crRNA and tracrRNA. Examples of such crRNA and tracrRNA are described in WO 2016/100951 A2 (Integrated DNA Technologies, Inc., U.S.A.). The tracrRNA may, for example have a length of 67 nt and have the following sequence (SEQ ID NO: 5) 5-agcauagcaaguuaaaauaaggcuaguccguuaucaacuugaaaaaguggcaccgagucggugcuuu-3 (see WO WO 2016/100951 A2), and the crRNA may have a length of 36 nt and the following sequence (SEQ ID NO: 6): 5-nnnnnnnnnnnnnnnnnnnnguuuuagagcuaugcu-3, n being any nucleotide, depending on the target DNA.

Preferably, in the method according to the invention the tracrRNA is labeled with a fluorescence label, preferably at the 5' terminus. The label can, however, be any suitable label, e.g. an isotopic label, radiolabel, or a nanoparticle label, e.g. gold nanoparticle label.

In the method of the invention the sample can not only be contacted with a single type of ribonucleoprotein complexes, but can additionally be simultaneously contacted with at least one or more further different ribonucleoprotein complexes. The at least two different ribonucleoprotein complexes differ at least in their crRNA, such that different target DNA sequences in the sample can be targeted, and preferably additionally in the label of the tracrRNA, such that the different ribonucleoprotein complexes are labeled with different labels, preferably different fluorescence labels. Preferably, the tracrRNAs of the different ribonucleoprotein complexes each have a different label, e.g. fluorescence label. In this embodiment of the method different target DNA sequences can, for example, be visualized by using different fluorescence labels having different emission wavelengths, i.e. colors.

In the method of the invention the paraformaldehyde-fixed tissue section is contacted with a buffered 4% paraformaldehyde solution after being contacted with the ribonucleoprotein complex. It has been found that this additional post-fixation step may be useful for preventing dissociation of a Cas protein from the target DNA. This step is performed under cooling, e.g. on ice.

In one embodiment, the method of the invention comprises the steps of:
a) incubating a tissue sample fixed with buffered 4% paraformaldehyde solution with the ribonucleoprotein complex at a temperature of 4-37 °C, preferably 15-30 °C, further preferred 20-28°C, especially 25-26 °C, wherein the tracrRNA is labeled with a fluorescence label,
b) incubating the tissue sample of step a) with buffered 4% paraformaldehyde solution on ice, and
c) measuring the fluorescence in the sample.

In the method of the invention, a detection of labeled DNA target sequences is already possible after approximately 10 seconds.

The method can also be modified to simultaneously detect proteins, for example DNA binding proteins, in the sample. The method of the invention may thus additionally comprise the step of further contacting the sample with a labeled antibody, preferably a fluorescently labeled antibody. The antibody may be any antibody binding to a suitable target antigen, such as a protein. The antibody may, for example, be directed against a DNA binding protein.

Preferably a fluorescently labeled tracrRNA and a fluorescently labeled antibody are used in the method, and the fluorescence labels of the tracrRNA and of the antibody preferably have different emission wavelengths in order to distinguish DNA signals from protein signals.

In a preferred embodiment the method of the invention is an in-situ method.

In the following, the invention is described by way of attached figures and examples for illustration purposes only.

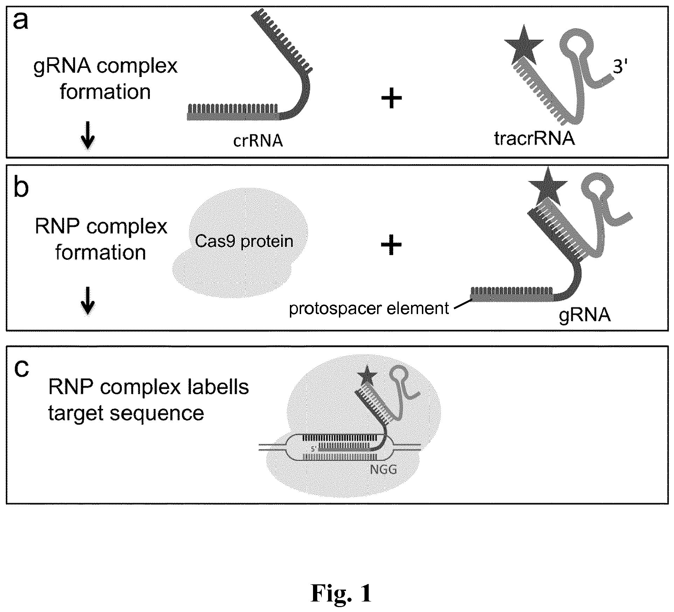

Figure 1. Diagram of the RGEN-ISL method. (a) Guide RNA (gRNA) complex formation after hybridization of CRISPR RNA (crRNA) and 5' ATTO 550 (star) labeled trans-activating crRNA (tracrRNA). (b) Ribonucleoprotein (RNP) complex formation after combination of recombinant Cas9 protein with gRNA. (c) Components of the RGEN-ISL system to label genomic targets. The crRNA:tracrRNA complex uses optimized Alt-R crRNA and ATTO 550-labelled tracrRNA sequences that hybridize and then form a complex with Cas9 endonuclease to guide targeted binding to genomic DNA. The binding site is specified by the protospacer element of the crRNA. The crRNA protospacer element recognizes 19 or 20 nt on the opposite strand of the NGG PAM site. The PAM site must be present immediately downstream of the protospacer element for binding to occur.

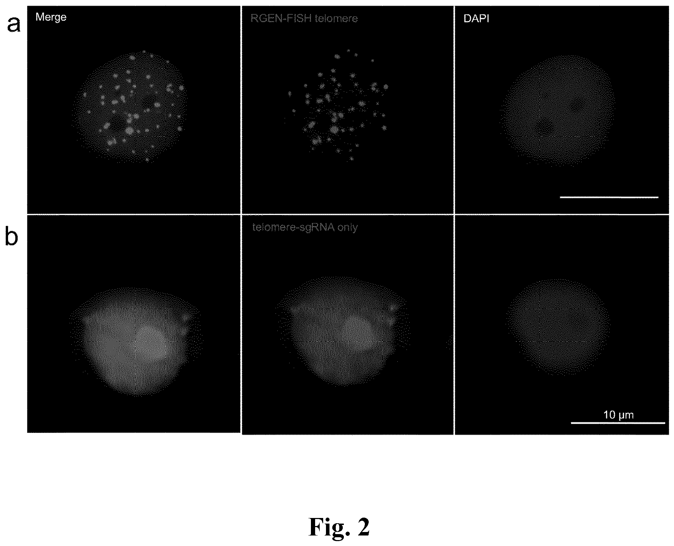

Figure 2. RGEN-ISL with *Arabidopsis-type* telomere-specific guide RNA. (a) Nucleus of *N. benthamiana* showing telomere-typical dot-like signals distributed over the entire nucleus. (b) Application of ATTO 550 labelled guide RNA without the Cas9 protein as a negative control. RGEN-FISH = RGEN-ISL

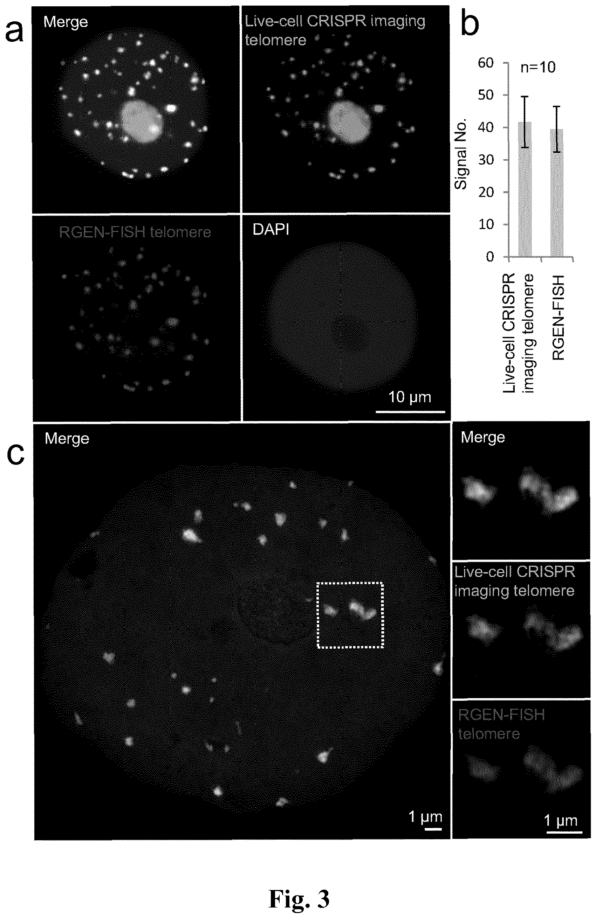

Figure 3. RGEN-ISL detects specific sequences. (a) Nucleus of *N. benthamiana* exhibiting telomere-specific live-cell CRISPR imaging signals after subsequent telomere-specific RGEN-ISL. (b) Number of telomere live-cell CRISPR and REGEN-FISH signals per nucleus (n=10 nuclei). No significant difference was found (p=5%). (c) SIM demonstrates a similar arrangement of live-cell CRISPR-imaging and RGEN-ISL signals. Insets show a further enlarged subnuclear region exhibiting telomere signals. RGEN-FISH = RGEN-ISL

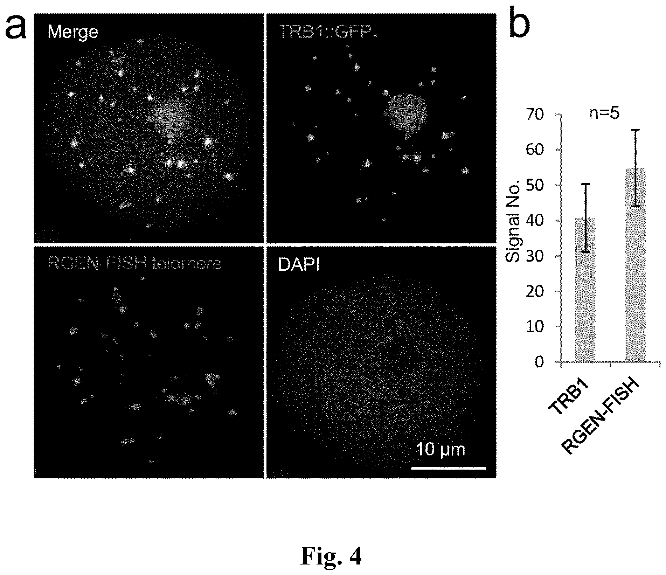

Figure 4. RGEN-ISL in combination with fluorescently tagged proteins allows double labelling of DNA and endogenous proteins. (a) Nucleus of N. benthamiana exhibiting TRB1::GFP signals after subsequent telomere-specific RGEN-ISL. (b) Number of TRB1::GPF and telomere-specific RGEN-ISL signals per nucleus (n=5 nuclei). No significance in 5%. RGEN-FISH = RGEN-ISL, TRB1 = Telomere repeat-binding factor 1, GFP = Green Fluorescent Protein

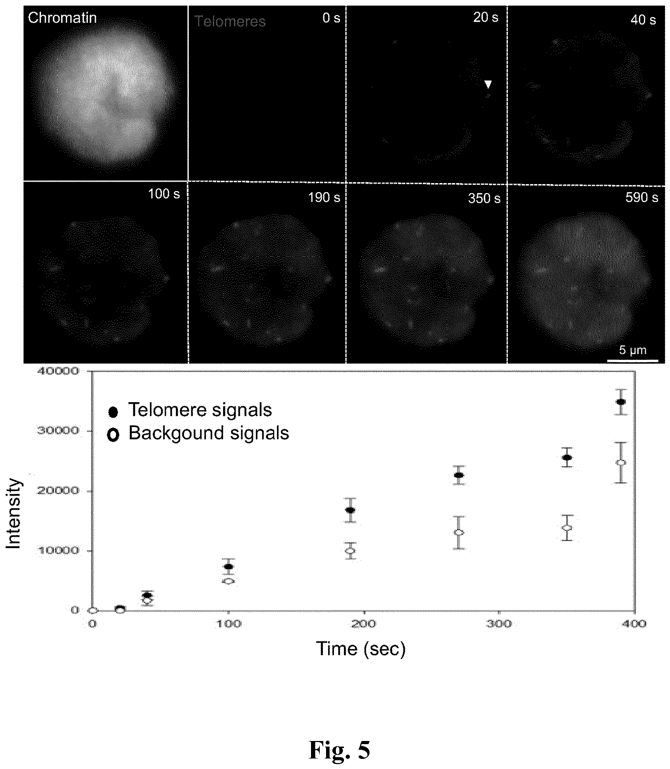

Figure 5. Time-lapse microscopy demonstrates the dynamics of the dCas9-RNA complex to label the telomeres of fixed *N. benthamiana* nuclei. (a) First telomere signals (white arrow head) are visible 20 seconds after applying the RNP complex. Nucleus is counterstained with DAPI. (b) The telomere as well as the background increase in the course of time. The intensities of 17 telomere signals at 8 different times were measured from 0 to 390 seconds. In parallel we determined the background intensity at 3 different sites outside the telomeres. For each time we calculated the mean and standard error of mean.

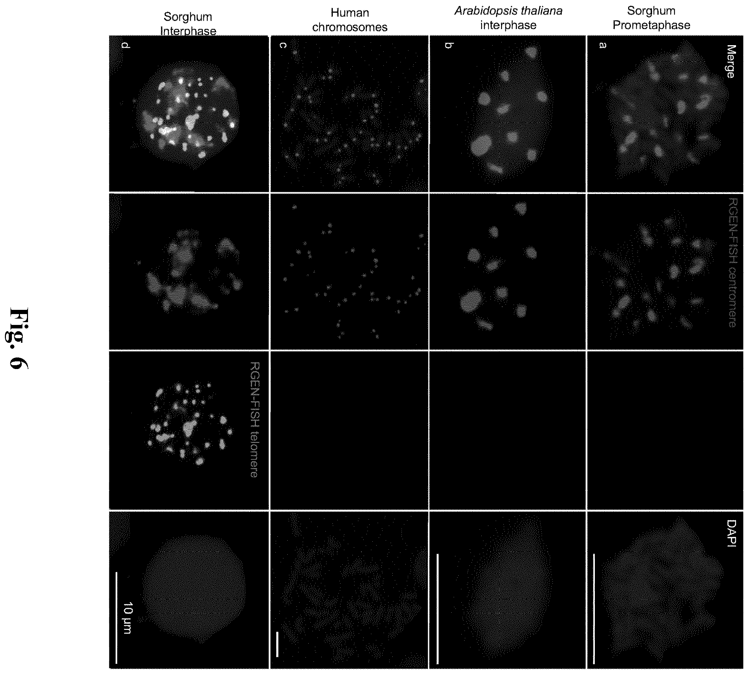

Figure 6. RGEN-ISL for centromeres in different species and multicolor RGEN-ISL. (a-c) RGEN-ISL based detection of centromere repeats in *Sorghum, Arabidopsis thaliana* and *Homo sapiens.* (d) Multicolor RGEN-ISL allows the simultaneous detection of telomere and centromere repeats in Sorghum. RGEN-FISH = RGEN-ISL

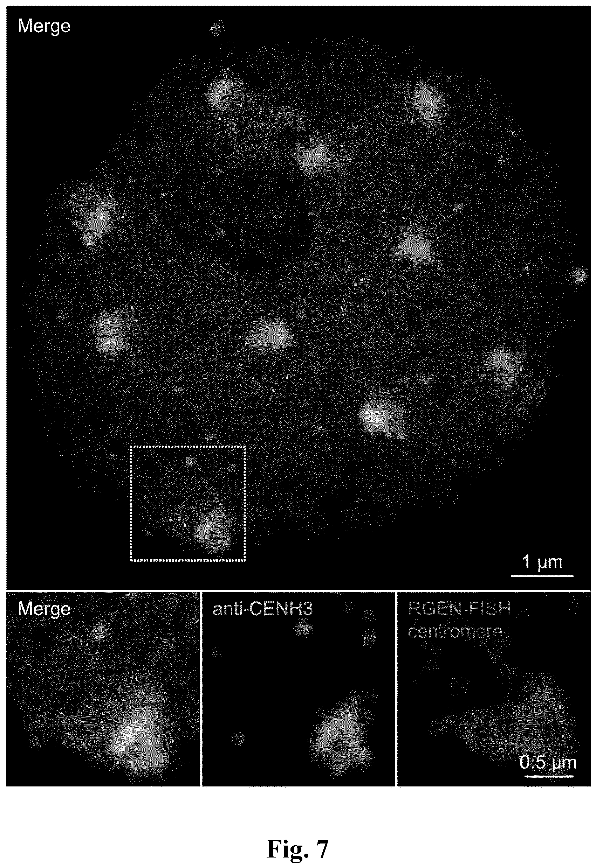

Figure 7. RGEN-ISL in combination with immunostaining allows simultaneous labelling of DNA and endogenous proteins. *Arabidopsis* interphase nucleus showing anti-CENH3 and centromere-specific RGEN-ISL signals. Insets show a further enlarged subnuclear region exhibiting centromeric signals. RGEN-FISH = RGEN-ISL, CENH3 = centromere-specific histone H3

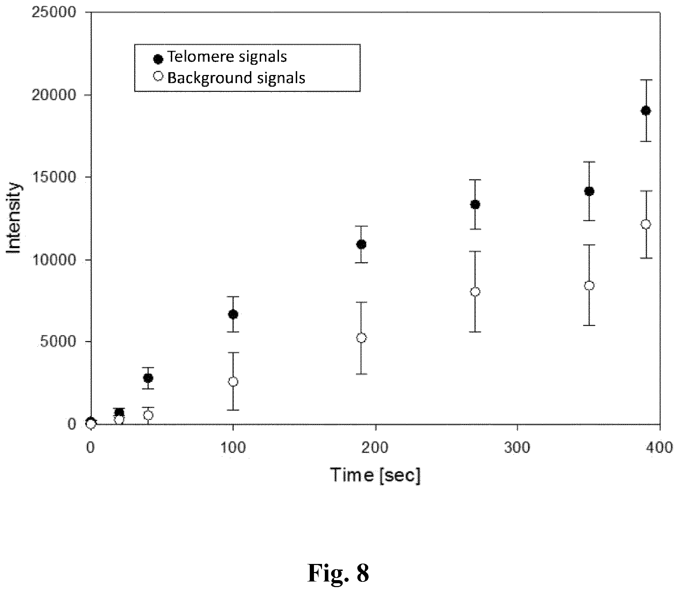

Figure 8. Time-lapse microscopy demonstrates the dynamics of the dCas9-RNA complex to label the telomeres of fixed *N. benthamiana* nuclei. The telomere as well as the background signals increase in the course of time. The intensities of 12 telomere signals at 8 different times from 0 to 390 seconds. In parallel the background intensity was determined at 3 different sites outside the telomeres. For each time we calculated the mean and standard error of mean. Mean telomere signal (●) and background (ο) intensities ± standard errors of mean.

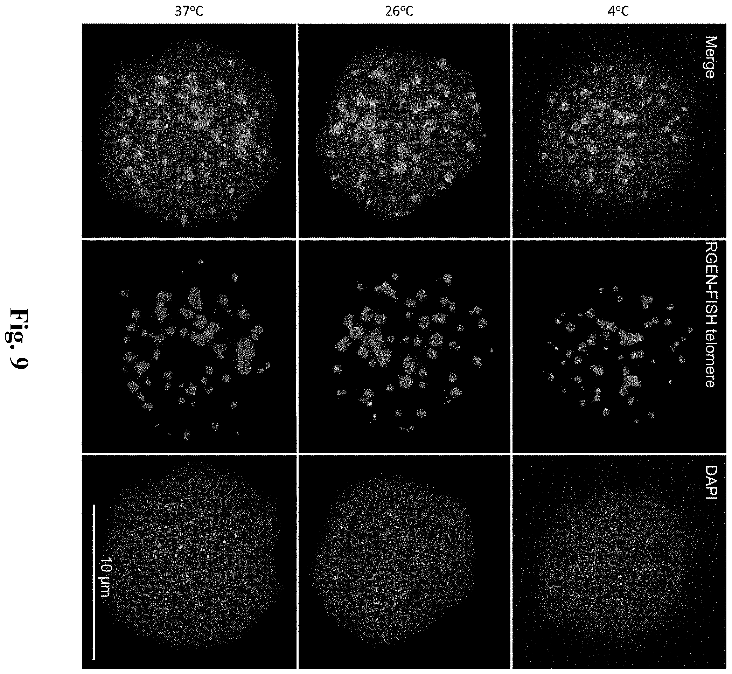

Figure 9. An incubation temperature between 4°C - 37°C is suitable for RGEN-ISL. Telomere-specific RGEN-ISL was performed under identical conditions (1 hour incubation). except different temperatures (4°C, 26°C and 37°C). The telomere signals of *N. benthamiana* nuclei were recorded with the same CCD camera setting (200 msec exposure time in all cases). RGEN-FISH = RGEN-ISL

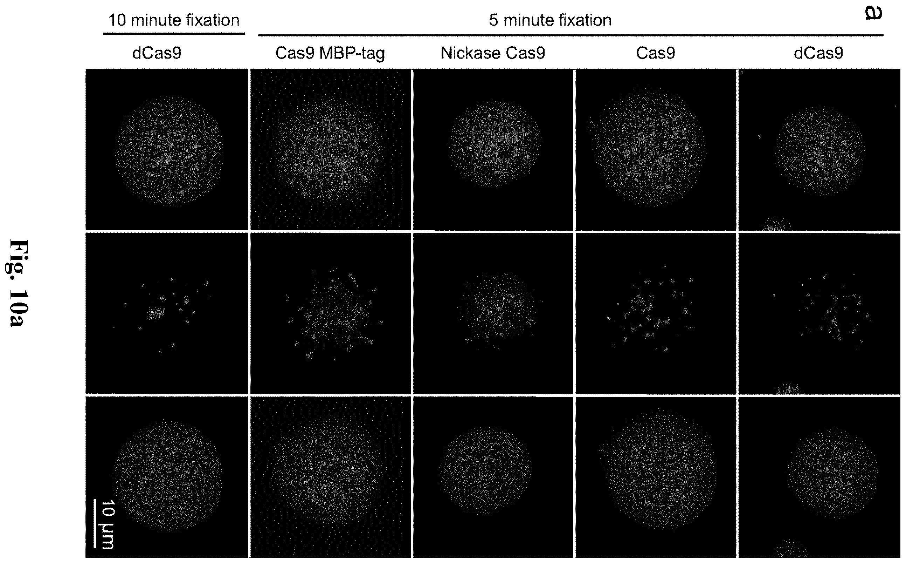

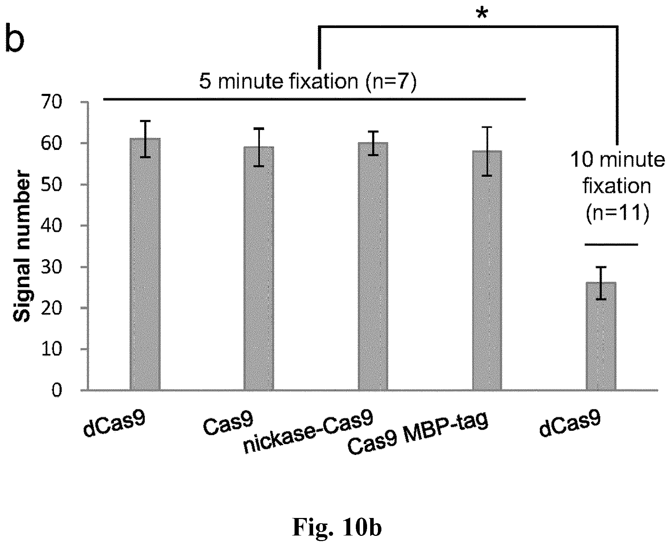

Figure 10. Comparison of different types of Cas9 and fixation condition for RGEN-ISL. (a) Nuclei of *N. benthamiana* showing telomere signals irrespective of the cutting activity of the Cas9 (nuclease-deficient Cas9 (dCas9), active Cas9, Cas9 with a MBP-tag or Cas9 nickase) via 5 or 10 minute vacuum with 4% formaldehyde fixation. (b) Telomere signals are significantly reduced when using a 10 minute vacuum with 4% formaldehyde-fixation. ^{∗} indicate 5% significance. RGEN-FISH = RGEN-ISL

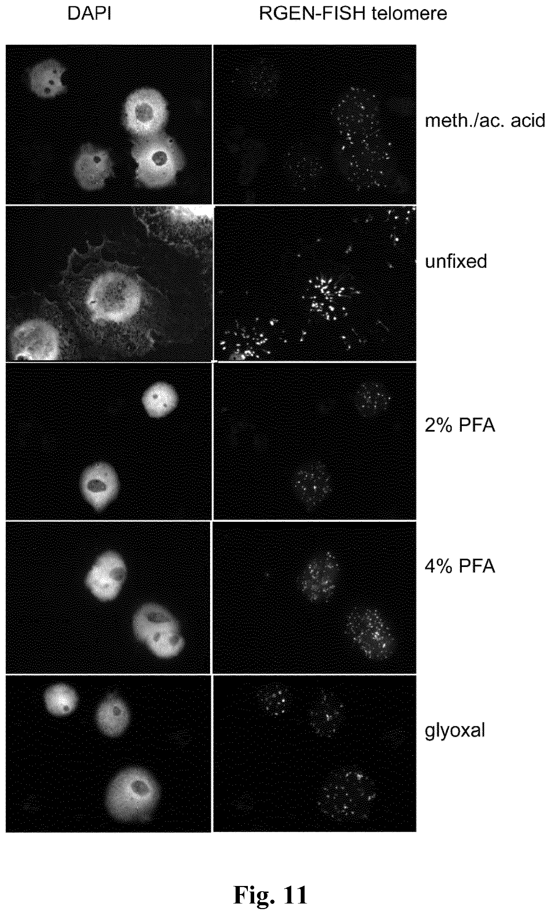

Figure 11. Influence of fixation method on RGEN-ISL based telomere signals. Before nuclei isolation and RGEN-ISL, leaf tissue of *N. benthamiana* was fixed with either 20°C prechilled solution of methanol/acetic acid at a 1:1 ratio (meth./ac. acid), 2% solved paraformaldehyde (PFA), 4% solved paraformaldehyde (PFA) or glyoxal (Richter et al., 2018). Note: unfixed tissue resulted in the strongest RGEN-ISL signals.

For the embodiments of the method of the invention described in the following, the terms "RGEN-ISL" or "RGEN-FISH" are used here. RGEN-ISL stands for RNA-guided endonuclease in situ labeling, RGEN-FISH for RNA-guided endonuclease fluorescent in situ hybridization.

In the following, the method of the invention is described in terms of the labeling of fixed plant and human nuclei and chromosomes. In addition to Cas9 from *S. pyogenes,* other RNA-guided endonucleases also have the potential to directly visualize genomic loci or RNA (Abudayyeh et al., 2017). The method of the invention preserves the natural spatio-temporal organization of the chromatin and allows the specific and simultaneous in situ detection of multi-colored genomic sequences by applying a complex of two-part guide RNA and recombinant Cas9 endonuclease. Moreover, the method is combinable with immunohistochemistry.

### Material and methods

### Material

Nuclei and chromosomes of *Nicotiana benthamiana, Arabidopsis thaliana, Sorghum bicolor,* and *Homo sapiens.*

### Methods

### Preparation of recombinant SpCas9

Cas9 *of S. pyogenes* encoded on vector pMJ806 (Addgene plasmid # 39312) (Jinek et al., 2012) was expressed in *E. coli* strain BL21 Rosetta 2 (DE3) (Novagen). Clarified cell lysate was bound to a 5 ml HisTrap FF (GE Healthcare). After buffer exchange, the 6x His-MBP Tag was removed by TEV protease cleavage. Protein and cleaved fusion tag were separated on a 5 ml HiTrap SP column (GE Healthcare). After a final size exclusion chromatography step on a HiLoad 16/600 Superdex 200 column (GE Healthcare) in 30 mM HEPES pH 7.5, 200 mM NaCl ,2 mM MgCl₂ and 1 mM TCEP, the protein was concentrated to ~6 mg/ml, flash frozen in liquid nitrogen and stored at -80°C.

### Formation of the ribonucleoprotein

For gRNA formation lyophilized crRNA and tracrRNA-ATTO 550/Alexa Fluor 488 was prepared in nuclease-free duplex buffer to 100 µM (IDT, https://eu.idtdna.com). Dissolved crRNA and tracrRNA can be stored separately at -20oC. crRNA and ATTO 550 labelled tracrRNA (Alt-R CRISPR-Cas9 system) are products of IDT (Integrated DNA Technologies, https://eu.idtdna.com/site). Protospacer sequences were selected based on the respective protospacer adjacent motif (PAM) sequence of S. pyogenes Cas9 (Table 1). To assemble 10 µM gRNA : 1 µl 100 µM crRNA, 1 µl 100 µM tracrRNA-ATTO 550/Alexa Fluor 488 was mixed with 8 µl duplex buffer. After, the gRNA was denatured for 5 min at 95 °C and allowed to hybridize. gRNA is stable at ―20 °C until use. For RNP complex assembly 1 µl 10 µM sgRNA, 1 µl 6.25 µM dCas9 proteins ((D10A *&* H840A) cat nr: PR-137213, Novateinbio,www.novateinbio.com), 10 µl 10x Cas9 buffer (Deng et al., 2015), 10 µl 10 mM DTT and 80 µl double distilled water were mixed and incubated at 26 °C for 10 min and stored at 4 °C. 100 µl of the RNP complex are enough for 3-4 slides. Instead of dCas9, the following Cas9 variants can be used: Recombinant SpCas9 with MBP-tag or without MBP-tag described above, active Cas9 (Alt-R® S.p. Cas9 Nuclease, 3NLS) and Nickase Cas9 (Alt-R® S.p. Cas9 D10A Nickase, IDT).

**Table1. crRNA sequences used for RGEN-ISL.**

| Name | Sequence |
|---|---|
| Sorghum centromere | 5'-AUUAGGAUAAGAAACCAUUU-3' (SEQ ID NO: 1) |
| Arabidopsis-type telomere | 5' GGGUUUAGGGUUUAGGGUUU 3' (SEQ ID NO: 2) |
| Arabidopsis centromere | 5'-UUGAGAAGCAAGAAGAAGGU-3' (SEQ ID NO: 3) |
| Human centromere | 5'-AGAAUCUGCAAGUGGAUAUU-3' (SEQ ID NO: 4) |

### Preparation of nuclei and chromosomes for RGEN-ISL

Leaf tissue was fixed in ice-cold 4% formaldehyde in Tris-buffer (10 mM Tris-HCL, (pH 7.5), 10 mM Na₂-EDTA, 100 mM NaCl, 0.1% Triton X-100, adjust to pH 7.5 with NaOH) for 5 min under vacuum Fixation continued for 25 min in ice cold fixative without vacuum. Tissue was rinsed 2x 5 min in ice-cold Tris-buffer. Tissue was chopped in 400-500 µl ice-cold LB01 buffer as described (Dolezel et al., 1989). LB01-buffer: 15 mM Tris-HCl, (pH 7.5), 2 mM Na₂-EDTA, 0.5 mM Spermin, 80 mM KCl, 20 mM NaCl, 15 mM β-Mercaptoethanol, 0.1% Triton X-100.

The suspension was filtered through a mesh of 35 µm pore size and spun onto glass slides using a cytospin (700 rpm for 5 min). After, slides were kept in ice cold 1x phosphate buffered saline (PBS) until use.

### RGEN-ISL procedure

For each slide, 100 µl of 1x Cas9/1mM DTT buffer (10x Cas9 buffer: 200 mM Hepes (pH 7.5), 1M KCl, 50 mM MgCl₂, 50% (vol/vol) glycerol, 10% BSA and 1% TWEEN-20) was applied for 2 min at room temperature (RT). Then, slides were tilted to remove the buffer and 20 - 30 µl RNP complex per slide were added. Slides were covered with parafilm and kept in a moisture chamber at 26 °C for 1-4 h. Note: Incubation time is species and sequences dependent. In addition, incubation at 4°C overnight is possible. After incubation slides were washed in ice-cold 1x PBS for 5 min. Note: In case of strong background signals extend washing time. To prevent the dissociation of the RNP complex, post-fixation was performed with 4% formaldehyde in 1x PBS for 5 min on ice. Then, slides were washed with 1x PBS for 5 min on ice and dehydrate with ethanol (70%, 90%, 96%; 2 min each) at room temperature. Slides were counterstained with 4',6-diamino-2-phenylindole (DAPI) and mounted in Vectashield mounting medium (Vector Laboratories, Burlingame, CA, USA).

### Combination of immunostaining and RGEN-ISL

Slides were prepared as described for RGEN-ISL. 60-70 µl of primary antibody (e.g. 1:1000 diluted in 1x PBS anti-AtCENH3 (Talbert, 2002) was applied per slide and incubated at 4 °C overnight in a humid box. Slides were washed in 1x PBS for 2x 5 minute on ice. Then, 100 µl of 1x Cas9 buffer/1 mM DTT was applied per slide for 2 min at room temperature. After, the buffer was replaced by the secondary antibody (e.g. 1:500 dilution, Alexa Fluor 488 goat anti-rabbit IgG, Thermo Fisher) containing the prepared RNP complex. 20-30 µl of the RNP complex including the diluted secondary antibody were applied per slide. Slides were covered with parafilm and kept in a moisture chamber at 37 °C for one hour. Then, the slides were washed in ice-cold 1x PBS for 5 min. Post-fixation and subsequent steps were performed as described for RGEN-ISL.

### Transient transformation of N. benthamiana

The Sp-dCas9-eGFP construct to label telomeres (Dreissig et al., 2017) and TRB1-GFP (Schrumpfova et al., 2014) were separately transformed into *Agrobacterium tumefaciens* strain GV3101 by electroporation. Transient transformation of *N. benthamiana* leaf cells was performed as described in (Phan and Conrad, 2016). Nuclei for subsequent RGEN-ISL were isolated 2-4 days after infiltration and used for RGEN-ISL.

### Microscopy

Classical fluorescence imaging was performed using an Olympus BX61 microscope equipped with an ORCA-ER CCD camera (Hamamatsu). All images were acquired in grey scale and pseudocolored with Adobe Photoshop 6 (Adobe Systems). To analyze the ultrastructure and spatial arrangement of signals and chromatin at a lateral resolution of~120 nm (super-resolution, achieved with a 488 nm laser), 3D structured illumination microscopy (3D-SIM) was applied using 63 ×/1.4 Oil Plan-Apochromat objective of an Elyra PS.1 microscope system and the software ZENblack (Carl Zeiss GmbH). Image stacks were captured separately for each fluorochrome using the 561, 488, and 405 nm laser lines for excitation and appropriate emission filters (Weisshart et al., 2016). Maximum intensity projections of whole nuclei were calculated via the ZEN software. Zoom in sections were presented as single slices to indicate the subnuclear chromatin and protein structures at the super-resolution level.

### RESULTS

A general scheme for producing a fluorescently labelled ribonucleoprotein complex (with Cas9 as an example for a Cas protein) is depicted in Figure 1. A two-part gRNA complex consisting of crRNA and a labeled tracrRNA is produced (Fig. 1a). The label is here represented by a star. This step takes about 10 min. The two-part gRNA complex is combined with the Cas protein in order to produce the RNP complex. This step also takes about 10 min.

To produce a fluorescently labelled ribonucleoprotein complex (RNP), we employed recombinant dCas9 protein *of S. pyogenes.* Because the synthesis of fluorochrome-coupled sgRNA probes (~100 nt) is costly, we decided to test a universally 5' ATTO™ 550 labeled 67 nt Alt-R® tracrRNA that contains proprietary chemical modifications conferring increased nuclease resistance (Schubert et al., 2017). The synthetic 67 nt Alt-R tracrRNA and the 36 nt Alt-R-crRNA are much shorter than the native guide *of S. pyogenes* (89/42 nt), leading to an increased on-target performance (Jacobi et al., 2017). Alt-R tracrRNA hybridizes to the 16 nt complementary region of the crRNA to activate the Cas9 enzyme. The 35-36 nt crRNA, containing the 19 or 20 nt target specific protospacer element, recognizes the complementary strand of the NGG PAM site. Alt-R tracrRNA pairs with the target-specific crRNAs, thus allowing the easy and cost-effective studying of many gRNA specific sites (Figure 1).

To assay the functionality of RGEN-ISL, we imaged the telomeres in formaldehyde-fixed nuclei of *Nicotiana benthamiana. N. benthamiana* telomeres are composed of 60-160 kb-long arrays of TTTAGGG repeats (Fajkus et al., 1995). These tandemly repeated DNA sequences allow the binding of many Cas9 proteins at the same locus by a single crRNA sequence. To target telomeric repeats, we selected a 20 nucleotide-long crRNA complementary to the TTTAGGG telomere sequence starting with a 'G' at the 5'-end as previously used for live cell CRISPR-imaging (Dreissig et al., 2017). Bright fluorescence dots were observed in the nuclei (Figure 2a). Performing the same experiment without the Cas9 protein as a negative control resulted in only a diffuse unspecific labelling of the entire nuclei (Figure 2b).

To confirm the specificity and efficiency of the RGEN-ISL method we repeated our experiment on nuclei of *N. benthamiana* exhibiting telomere-specific dCas9::GFP signals (Dreissig et al., 2017). A clear overlap of both types of telomere signals was found (Figure 3a, b). The on average 39.5 telomere RGEN-ISL signals indicate a certain degree of telomere clustering since the expected number of telomeres based on a chromosome complement of 2n = 38 would be 76 in 2C nuclei.

To determine the subnuclear localization of both types of RNA-guided endonuclease-based DNA imaging probes, we employed structured illumination microscopy (SIM) to achieve a resolution of up to 120 nm. We found a similar arrangement and intensity of RGEN-ISL and live-cell CRISPR-imaging signals (Figure 3c). Hence, both methods have a similar labelling efficiency. The ability of RGEN-ISL to label the same genomic loci which were already detected by live-cell CRISPR-imaging suggests that only a subset of telomere repeats were detected by the live-cell CRISPR imaging reporter.

In addition, we labelled fixed nuclei expressing the GFP-fused telomere repeat binding protein 1 (TRB1) (Figure 4). TRB1 locates at telomeres and interacts with the telomere reverse transcriptase, although not all telomeres are bound by TRB1 (Dvorackova et al., 2010; Schrumpfova et al., 2014). Interestingly, telomeres in yeast, ciliates, mammals and plants may form 3' overhangs. However, in plants, blunt-ended telomeres and telomeres with 3' overhangs may appear in the same cell and only telomeres exhibiting 3' overhangs are bound by TRB1. On average, we detected 50 RGEN-ISL signals resembling telomeres out of which 40 (87.6%) were simultaneously bound by TRB1 (Figure 4a, b). This indicates that most telomeres in N. benthamiana form 3' overhangs and only a small proportion of blunt-ended telomeres is present during interphase. Our results demonstrate that RGEN-ISL can also be used to visualize specific DNA sequences in combination with fluorescently tagged endogenous proteins interacting with those DNA sequences.

The Cas9-RNA complex interrogates the target sites on the DNA via three-dimensional diffusion, and recognizes the complementary target site with the NGG PAM diffusion (Shibata et al., 2017). To visualize the real-time dynamics of the dCas9-RNA complex to label telomeres of fixed nuclei in action time-lapse microscopy was employed (Figure 5, Figure 8). Notably, only 20 seconds after applying the pre-assembled dCas9-RNA complex, the first telomere signals became detectable. The intensity of fluorescence signals steadily increased during the next 590 seconds. Background signals increased in the course of time too, as no washing step was included. The accumulation of the telomere signal intensity suggests, in line with previous biochemical experiments (Sternberg et al., 2014), that the dCas9-RNA complex remains tightly bound to the DNA after target identification.

Although, we noted that incubation temperatures between 4°C and 37°C are suitable for this method, we used 26°C for most experiments (Figure 9). To stabilize the fluorescence signals after the RGEN-ISL reaction, a short post-fixation step in 4% formaldehyde on ice is advantageous to prevent a dissociation of the dCas9-RNA complex from DNA. Besides the application of the nuclease-deficient dCas9 protein, recombinant active Cas9, Cas9 with an MBP-tag or Cas9 nickase were also successfully used for RGEN-ISL (Figure 10a). Irrespective of the cutting ability, all Cas9 variants resulted in a similar number (no significant difference in p= 5%) and intensity of telomere signals. For most of the RGEN-ISL experiments, we used a 5 minute vacuum with 4% formaldehyde-fixed samples. An extended formaldehyde-based fixation of tissues reduced the efficiency of the RGEN-ISL method, likely by blocking the ribonucleoprotein complex access to the target sequence (Figure 10a, b). On the other hand, methanol/acetic acid-based fixation of tissue as used by Deng et al., 2015 leads to a poor morphology preservation of nuclei in plant (Figure 11). Application of unfixed nuclei resulted in the strongest RGEN-ISL signals, but was combined with destructed nuclei. Further, fixation of tissues in glyoxal only (Richter et al., 2018) did not perform better than formaldehyde (Figure 11).

Besides the detection of telomeres, we demonstrated that RGEN-ISL allows the detection of centromeric tandem repeats in *Sorghum, Arabidopsis thaliana* and *Homo sapiens* (Figure 6a, b and c respectively). Further, the simultaneous application of 5' Alexa fluor® 488 and 5' ATTO™ 550 labelled tracrRNAs paved the way to differentially label multiple genomic loci including the centromeres and telomeres of *Sorghum* (Figure 6d).

Since RGEN-ISL is a tool to visualize specific DNA sequences in non-denatured chromatin, it can be combined with protein-based detection methods, e.g. immunofluorescence, to study the colocalization of DNA-protein complexes. As an example, we attempted to simultaneously visualize centromeric DNA by RGEN-ISL and the centromere-specific histone H3 variant CENH3 by immunostaining of *A. thaliana* (Figure 7). A fast detection of specific DNA and proteins is possible, as RGEN-ISL and immunostaining work in the same buffer. We hypothesize that this principle can be expanded to investigate spatio-temporal gene expression patterns, e.g. by visualizing DNA sequences and histone variants.

One limitation of RGEN-ISL using Cas9 and other type II Cas proteins is the requirement of the presence of a protospacer adjacent motif (PAM) at the target site. For the most commonly used Cas9 from S. pyogenes, the required PAM sequence is NGG. To overcome this limitation, natural or engineered Cas9 variants having different PAM sequence requirements can be used (Hu et al., 2018; Ma et al., 2015). For the detection of tandem repetitive sequences, a single gRNA is sufficient for RGEN-ISL imaging. However, the detection of non-repetitive loci may require a simultaneous application of multiple gRNAs as demonstrated for the CRISPR-based detection of a 5 kb region in living mammalian cells (Chen et al., 2013).

### References

Abudayyeh, O.O., Gootenberg, J.S., Essletzbichler, P., Han, S., Joung, J., Belanto, J.J., Verdine, V., Cox, D.B.T., Kellner, M.J., Regev, A., et al. (2017). RNA targeting with CRISPR-Cas13. Nature 550, 280-+.
Ahmad, H.I., Ahmad, M.J., Asif, A.R., Adnan, M., Iqbal, M.K., Mehmood, K., Muhammad, S.A., Bhuiyan, A.A., Elokil, A., Du, X., et al. (2018). A Review of CRISPR-Based Genome Editing: Survival, Evolution and Challenges. Curr Issues Mol Biol 28, 47-68.
Anton, T., Bultmann, S., Leonhardt, H., and Markaki, Y. (2014). Visualization of specific DNA sequences in living mouse embryonic stem cells with a programmable fluorescent CRISPR/Cas system. Nucleus 5, 163-172.
Boettiger, A.N., Bintu, B., Moffitt, J.R., Wang, S., Beliveau, B.J., Fudenberg, G., Imakaev, M., Mirny, L.A., Wu, C.T., and Zhuang, X. (2016). Super-resolution imaging reveals distinct chromatin folding for different epigenetic states. Nature 529, 418-422.
Chen, B., Gilbert, L.A., Cimini, B.A., Schnitzbauer, J., Zhang, W., Li, G.W., Park, J., Blackburn, E.H., Weissman, J.S., Qi, L.S., et al. (2013). Dynamic imaging of genomic loci in living human cells by an optimized CRISPR/Cas system. Cell 155, 1479-1491.
Deng, W., Shi, X., Tjian, R., Lionnet, T., and Singer, R.H. (2015). CASFISH: CRISPR/Cas9-mediated in situ labeling of genomic loci in fixed cells. Proc Natl Acad Sci U S A 112, 11870-11875.
Dolezel, J., Binarova, P., and Lucretti, S. (1989). Analysis of Nuclear-DNA Content in Plant-Cells by Flow-Cytometry. Biol Plantarum 31, 113-120.
Dreissig, S., Schiml, S., Schindele, P., Weiss, O., Rutten, T., Schubert, V., Gladilin, E., Mette, M.F., Puchta, H., and Houben, A. (2017). Live-cell CRISPR imaging in plants reveals dynamic telomere movements. Plant J 91, 565-573.
Dvorackova, M., Rossignol, P., Shaw, P.J., Koroleva, O.A., Doonan, J.H., and Fajkus, J. (2010). AtTRB1, a telomeric DNA-binding protein from Arabidopsis, is concentrated in the nucleolus and shows highly dynamic association with chromatin. Plant J 61, 637-649.
Fajkus, J., Kovarik, A., Kralovics, R., and Bezdek, M. (1995). Organization of Telomeric and Subtelomeric Chromatin in the Higher-Plant Nicotiana-Tabacum. Molecular & General Genetics 247, 633-638.
Haft DH, Selengut J, Mongodin EF, Nelson KE (2005) A Guild of 45 CRISPR-Associated (Cas) Protein Families and Multiple CRISPR/Cas Subtypes Exist in Prokaryotic Genomes. PLOS Computational Biology 1(6): e60. doi:10.1371/journal.pcbi.0010060
Hu, J.H., Miller, S.M., Geurts, M.H., Tang, W., Chen, L., Sun, N., Zeina, C.M., Gao, X., Rees, H.A., Lin, Z., et al. (2018). Evolved Cas9 variants with broad PAM compatibility and high DNA specificity. Nature 556, 57-63.
Jacobi, A.M., Rettig, G.R., Turk, R., Collingwood, M.A., Zeiner, S.A., Quadros, R.M., Harms, D.W., Bonthuis, P.J., Gregg, C., Ohtsuka, M., et al. (2017). Simplified CRISPR tools for efficient genome editing and streamlined protocols for their delivery into mammalian cells and mouse zygotes. Methods 121, 16-28.
Jinek, M., Chylinski, K., Fonfara, I., Hauer, M., Doudna, J.A., and Charpentier, E. (2012). A Programmable Dual-RNA-Guided DNA Endonuclease in Adaptive Bacterial Immunity. Science 337, 816-821.
Kozubek, S., Lukasova, E., Amrichova, J., Kozubek, M., Liskova, A., and Slotova, J. (2000). Influence of cell fixation on chromatin topography. Analytical biochemistry 282, 29-38. Koonin EV, Makarova KS, and Zhang F. (2017). Diversity, classification and evolution of CRISPR-Cas systems. Current opinion in microbiology 37:67-78. doi:10.1016/j.mib.2017.05.008.
Ma, H.H., Naseri, A., Reyes-Gutierrez, P., Wolfe, S.A., Zhang, S.J., and Pederson, T. (2015). Multicolor CRISPR labeling of chromosomal loci in human cells. P Natl Acad Sci USA 112, 3002-3007.
Makarova, K. S., Wolf, Y. I., Alkhnbashi, O. S., Costa, F., Shah, S. A., Saunders, S. J., ... Koonin, E. V. (2015). An updated evolutionary classification of CRISPR―Cas systems. Nature Reviews. Microbiology, 13(11), 722-736. http://doi.org/10.1038/nrmicro3569
Phan, H.T., and Conrad, U. (2016). Plant-Based Vaccine Antigen Production. Methods Mol Biol 1349, 35-47.
Richter, K.N., Revelo, N.H., Seitz, K.J., Helm, M.S., Sarkar, D., Saleeb, R.S., D'Este, E., Eberle, J., Wagner, E., Vogl, C., et al. (2018). Glyoxal as an alternative fixative to formaldehyde in immunostaining and super-resolution microscopy. EMBO J 37, 139-159.
Schrumpfova, P.P., Vychodilova, I., Dvorackova, M., Majerska, J., Dokladal, L., Schorova, S., and Fajkus, J. (2014). Telomere repeat binding proteins are functional components of Arabidopsis telomeres and interact with telomerase. Plant J 77, 770-781.
Schubert, M.S., Cedrone, E., Neun, B., Behlke, M.A., and Dobrovolskaia, M.A. (2017). Chemical modification of CRISPR gRNAs eliminate type I interferon responses in human peripheral blood mononuclear cells. Journal of Cytokine Biology 3, 121.
Shibata, M., Nishimasu, H., Kodera, N., Hirano, S., Ando, T., Uchihashi, T., and Nureki, O. (2017). Real-space and real-time dynamics of CRISPR-Cas9 visualized by high-speed atomic force microscopy. Nat Commun 8, 1430. Sternberg, S.H., Redding, S., Jinek, M., Greene, E.C., and Doudna, J.A. (2014). DNA interrogation by the CRISPR RNA-guided endonuclease Cas9. Nature 507, 62-67.
Talbert, P.B. (2002). Centromeric Localization and Adaptive Evolution of an Arabidopsis Histone H3 Variant. The Plant Cell Online 14, 1053-1066.
Wang, H.F., La Russa, M., and Qi, L.S. (2016). CRISPR/Cas9 in Genome Editing and Beyond. Annu Rev Biochem 85, 227-264.
Weisshart, K., Fuchs, J., and Schubert, V. (2016). Structured illumination microscopy (SIM) and photoactivated localization microscopy (PALM) to analyze the abundance and distribution of RNA polymerase II molecules on flow-sorted Arabidopsis nuclei. Bio-protocol 6, e1725.
Wright AV, Nuñez JK, Doudna JA (January 2016). Biology and Applications of CRISPR Systems: Harnessing Nature's Toolbox for Genome Engineering. Cell. 164 (1-2): 29-44. doi:10.1016/j.cell.2015.12.035.
Zhang D, Chan S, Sugerman K, Lee J, Lam ET, Bocklandt S, Cao H, Hastie AR, CRISPR-bind: a simple, custom CRISPR/dCas9-mediated labeling of genomic DNA for mapping in nanochannel arrays, bioRxiv 371518; doi: https://doi.org/10.1101/371518.

## Claims

1. A method for the detection of a target DNA sequence in a sample, the sample being a tissue section which has been fixed with a buffered 4% paraformaldehyde solution, wherein the sample is contacted with a ribonucleoprotein complex comprising an unlabeled Cas protein, an unlabeled crRNA hybridizing with the target DNA sequence and a labeled tracrRNA hybridizing with the crRNA under conditions where the target DNA in the sample is able to hybridize with the crRNA, and wherein the paraformaldehyde-fixed tissue section is contacted with a buffered 4% paraformaldehyde solution under cooling after being contacted with the ribonucleoprotein complex.

2. The method according to claim 1, wherein the crRNA and the tracrRNA are separate RNA molecules.

3. The method according to any of claims 1 or 2, wherein the Cas protein is a recombinant Cas protein, preferably a recombinant type II Cas protein, further preferred a Cas protein lacking nuclease activity, especially preferred a nCas9 protein or dCas9 protein.

4. The method according to any of claims 2 or 3, wherein the crRNA and the tracrRNA are synthetic RNAs, and wherein the crRNA and the tracrRNA preferably are each shorter than naturally occurring crRNAs and tracrRNAs.

5. The method according to claim 4, wherein the tracrRNA has a length of 67 nt and a sequence of SEQ ID NO: 5, and the crRNA has a length of 36 nt and a sequence of SEQ ID NO: 6.

6. The method according to any of the preceding claims, wherein the tracrRNA is labeled with a fluorescence label or a gold nanoparticle label, preferably at the 5' terminus.

7. The method according to any of the preceding claims, wherein the sample is contacted with at least two different ribonucleoprotein complexes, wherein the ribonucleoprotein complexes differ at least in their crRNA and in the label of their tracrRNA, and wherein the different labels are preferably different fluorescence labels.

8. The method according to any of the preceeding claims, comprising the steps of:
a) incubating a tissue sample fixed with buffered 4% paraformaldehyde solution with the ribonucleoprotein complex at a temperature of 4-37 °C, preferably 15-30 °C, further preferred 20-28°C, especially preferred 25-26 °C, wherein the tracrRNA is labeled with a fluorescence label,
b) incubating the tissue sample of step a) with buffered 4% paraformaldehyde solution on ice, and
c) measuring the fluorescence in the sample.

9. The method according to any of the preceding claims, comprising the step of further contacting the sample with a labeled antibody, preferably a fluorescently labeled antibody.

10. The method according to claim 9, wherein the antibody is preferably directed against a DNA binding protein.

11. The method according to claim 9 or 10, wherein a fluorescently labeled tracrRNA and a fluorescently labeled antibody are used, and wherein the fluorescence labels of the tracrRNA and the antibody have different emission wavelengths.

12. The method according to any of the preceding claims, wherein the method is an in-situ method.

## Patentansprüche

1. Verfahren zum Nachweis einer Ziel-DNA-Sequenz in einer Probe, wobei die Probe ein Gewebeschnitt ist, der mit einer gepufferten 4% Paraformaldehydlösung fixiert wurde, und wobei die Probe mit einem Ribonukleoprotein-Komplex kontaktiert wird, umfassend ein unmarkiertes Cas-Protein, eine unmarkierte crRNA, die mit der Ziel-DNA-Sequenz hybridisiert, und eine markierte tracrRNA, die mit der crRNA unter Bedingungen hybridisiert, bei denen die Ziel-DNA in der Probe in der Lage ist, mit der crRNA zu hybridisieren, und wobei der Paraformaldehyd-fixierte Gewebeschnitt mit einer gepufferten 4% Paraformaldehydlösung unter Kühlung in Kontakt gebracht wird, nachdem er mit dem Ribonukleoproteinkomplex in Kontakt gebracht wurde.

2. Verfahren nach Anspruch 1, wobei die crRNA und die tracrRNA getrennte RNA-Moleküle sind.

3. Verfahren nach einem der Ansprüche 1 oder 2, wobei das Cas-Protein ein rekombinantes Cas-Protein, vorzugsweise ein rekombinantes Cas-Protein vom Typ II, weiter bevorzugt ein Cas-Protein ohne Nukleaseaktivität, besonders bevorzugt ein nCas9-Protein oder dCas9-Protein ist.

4. Verfahren nach einem der Ansprüche 2 oder 3, wobei die crRNA und die tracrRNA synthetische RNAs sind und wobei die crRNA und die tracrRNA vorzugsweise jeweils kürzer sind als natürlich vorkommende crRNAs und tracrRNAs.

5. Verfahren nach Anspruch 4, wobei die tracrRNA eine Länge von 67 nt und eine Sequenz gemäß SEQ ID NO: 5 aufweist und die crRNA eine Länge von 36 nt und eine Sequenz gemäß SEQ ID NO: 6 aufweist.

6. Verfahren nach einem der vorhergehenden Ansprüche, wobei die tracrRNA mit einer Fluoreszenzmarkierung oder einer Goldnanopartikel-Markierung, vorzugsweise am 5'-Terminus, markiert ist.

7. Verfahren nach einem der vorhergehenden Ansprüche, wobei die Probe mit mindestens zwei verschiedenen Ribonukleoprotein-Komplexen in Kontakt gebracht wird, wobei sich die Ribonukleoprotein-Komplexe zumindest in ihrer crRNA und in der Markierung ihrer tracrRNA unterscheiden, und wobei die unterschiedlichen Markierungen vorzugsweise unterschiedliche Fluoreszenzmarkierungen sind.

8. Verfahren nach einem der vorhergehenden Ansprüche, umfassend die Schritte des:
a) Inkubierens einer mit gepufferter 4% Paraformaldehydlösung fixierten Gewebeprobe mit dem Ribonukleoproteinkomplex bei einer Temperatur von 4-37 ° C, vorzugsweise 15-30 ° C, weiter bevorzugt 20-28 ° C, besonders bevorzugt 25-26 ° C, wobei die tracrRNA mit einer Fluoreszenzmarkierung markiert ist,
b) Inkubierens der Gewebeprobe aus Schritt a) mit gepufferter 4% Paraformaldehydlösung auf Eis, und
c) Messens der Fluoreszenz in der Probe.

9. Verfahren nach einem der vorhergehenden Ansprüche, umfassend den weiteren Schritt des Kontaktierens der Probe mit einem markierten Antikörper, vorzugsweise einem fluoreszenzmarkierten Antikörper.

10. Verfahren nach Anspruch 9, wobei der Antikörper vorzugsweise gegen ein DNAbindendes Protein gerichtet ist.

11. Verfahren nach Anspruch 9 oder 10, wobei eine fluoreszenzmarkierte tracrRNA und ein fluoreszenzmarkierter Antikörper verwendet werden, und wobei die Fluoreszenzmarkierungen der tracrRNA und des Antikörpers unterschiedliche Emissionswellenlängen aufweisen.

12. Verfahren nach einem der vorhergehenden Ansprüche, wobei das Verfahren ein In-situ-Verfahren ist.

## Revendications

1. Procédé de détection d'une séquence d'ADN cible dans un échantillon, l'échantillon étant une section de tissu qui a été fixée avec une solution de paraformaldéhyde à 4 % tamponnée, dans lequel l'échantillon est mis en contact avec un complexe ribonucléoprotéique comprenant une protéine Cas non marquée, un ARNcr non marqué s'hybridant avec la séquence d'ADN cible et un ARNtracr marqué s'hybridant avec l'ARNcr dans des conditions dans lesquelles l'ADN cible dans l'échantillon est apte à s'hybrider avec l'ARNcr, et dans lesquelles la section de tissu fixée dans du paraformaldéhyde est mise en contact avec une solution de paraformaldéhyde à 4 % tamponnée sous refroidissement après avoir été mise en contact avec le complexe ribonucléoprotéique.

2. Procédé selon la revendication 1, dans lequel l'ARNcr et l'ARNtracr sont des molécules d'ARN distinctes.

3. Procédé selon l'une quelconque des revendications 1 ou 2, dans lequel la protéine Cas est une protéine Cas recombinante, de préférence une protéine Cas de type II recombinante, de manière davantage préférée une protéine Cas ne présentant pas d'activité nucléase, notamment une protéine nCas9 ou une protéine dCas9.

4. Procédé selon l'une quelconque des revendications 2 ou 3, dans lequel l'ARNcr et l'ARNtracr sont des ARN synthétiques, et dans lequel l'ARNcr et l'ARNtracr sont de préférence chacun plus courts que les ARNcr et ARNtracr naturels.

5. Procédé selon la revendication 4, dans lequel l'ARNtracr a une longueur de 67 nt et une séquence de SEQ ID NO : 5, et l'ARNcr a une longueur de 36 nt et une séquence de SEQ ID NO : 6.

6. Procédé selon l'une quelconque des revendications précédentes, dans lequel l'ARNtracr est marqué avec un marqueur fluorescent ou un marqueur à nanoparticules d'or, de préférence au niveau de l'extrémité terminale 5'.

7. Procédé selon l'une quelconque des revendications précédentes, dans lequel l'échantillon est mis en contact avec au moins deux complexes ribonucléiques différents, dans lequel les complexes ribonucléiques diffèrent au moins par leur ARNcr et par le marqueur de leur ARNtracr, et dans lequel les différents marqueurs sont de préférence des marqueurs fluorescents.

8. Procédé selon l'une quelconque des revendications précédentes, comprenant les étapes suivantes :
a) incubation d'un échantillon de tissu, fixé avec une solution de paraformaldéhyde à 4 % tamponnée, avec le complexe ribonucléoprotéique à une température de 4 à 37 °C, de préférence de 15 à 30 °C, de manière davantage préférée de 20 à 28°C, de manière encore davantage préférée de 25 à 26 °C, dans lequel l'ARNtracr est marqué avec un marqueur fluorescent,
b) incubation de l'échantillon de tissu de l'étape a) avec une solution de paraformaldéhyde à 4 % tamponnée sur de la glace, et
c) mesure de la fluorescence dans l'échantillon.

9. Procédé selon l'une quelconque des revendications précédentes, comprenant l'étape de mise en contact ultérieure de l'échantillon avec un anticorps marqué, de préférence un anticorps marqué par fluorescence.

10. Procédé selon la revendication 9, dans lequel l'anticorps est de préférence dirigé contre une protéine de liaison à l'ADN.

11. Procédé selon la revendication 9 ou 10, dans lequel un ARNtracr marqué par fluorescence et un anticorps marqué par fluorescence sont utilisés, et dans lequel les marqueurs fluorescents de l'ARNtracr et de l'anticorps ont différentes longueurs d'onde d'émission.

12. Procédé selon l'une quelconque des revendications précédentes, dans lequel le procédé est un procédé *in situ.*


### SEQUENCE LISTING

<110> LEIBNIZ-INSTITUT FUER PFLANZENGENETIK UND KULTURPFLANZENFORSCHUNG
<120> RNA-GUIDED ENDONUCLEASE DNA LABELING
<130> PAT 1680 EP
<150> DE102018121111.2
   <151> 2018-08-29
<150> LU100916
   <151> 2018-08-29
<160> 6
<170> BiSSAP 1.3.6
<210> 1
   <211> 20
   <212> RNA
   <213> Artificial Sequence
<220>
   <223> crRNA
<400> 1
   auuaggauaa gaaaccauuu 20
<210> 2
   <211> 20
   <212> RNA
   <213> Artificial Sequence
<220>
   <223> crRNA
<400> 2
   ggguuuaggg uuuaggguuu 20
<210> 3
   <211> 20
   <212> RNA
   <213> Artificial Sequence
<220>
   <223> crRNA
<400> 3
   uugagaagca agaagaaggu 20
<210> 4
   <211> 20
   <212> RNA
   <213> Artificial Sequence
<220>
   <223> crRNA
<400> 4
   agaaucugca aguggauauu 20
<210> 5
   <211> 67
   <212> RNA
   <213> Artificial Sequence
<220>
   <223> tracrRNA
<400> 5
   agcauagcaa guuaaaauaa ggcuaguccg uuaucaacuu gaaaaagugg caccgagucg 60
   gugcuuu 67
<210> 6
   <211> 36
   <212> RNA
   <213> Artificial Sequence
<220>
   <223> crRNA
<220>
   <221> misc_feature
   <222> 1..20
   <223> /note="n = any nucleotide"
<400> 6
   nnnnnnnnnn nnnnnnnnnn guuuuagagc uaugcu 36